# EUROPEAN PATENT APPLICATION

(11) **EP 1 563 859 A1**
(43) Date of publication of application: **17.08.2005**
(21) Application number: 04292554.5
(22) Date of filing: 27.10.2004
(51) Int. Cl.: A61M 5/145, A61M 5/00

(54) **Liquid injection system having liquid injector capable of optically reading two-dimensional code assigned to liquid syringe**

(30) Priority: 29.10.2003 JP 2003368858
(71) Applicant: Nemoto Kyorindo Co., Ltd., Tokyo (JP)
(72) Inventor: Masuda, Kazumasa, c/o Nemoto Kyorindo Co., Ltd., Tokyo (JP)
(74) Representative: Burbaud, Eric

(57) **Abstract**

The liquid syringe (200) has various kinds of data items recorded in the two-dimensional code format (214). The liquid injector (100) optically reads the two-dimensional codes, decodes them, and executes a predetermined operations corresponding to the decoded results. Recording, for example, a variable pattern for the liquid of interest in the two-dimensional code format on the liquid syringe makes it possible for the liquid injector to inject the liquid in accordance with the predetermined variable pattern.

## Description

### BACKGROUND OF THE INVENTION

### 1. Field of the Invention

The present invention relates to a liquid injection system for injecting a liquid filled in a liquid syringe into a patient through a liquid injector, and particularly to a liquid injection system for injecting a contrast medium to a patient whose diagnostic image is to be taken through a diagnostic imaging apparatus such as a CT (Computed Tomography) scanner.

### 2. Description of the Related Art

A CT scanner, an MRI (Magnetic Resonance Imaging) apparatus, a PEP (Position Emission Tomography), an ultrasonic diagnostic apparatus, a CT Angio apparatus, an MRA (MR Angio) apparatus and the like are now used as a diagnostic imaging apparatus for shooting a diagnostic image of a patient. When using the above described diagnostic imaging apparatus, injection of a liquid such as the contrast medium or physiological saline has been made, and a liquid injector adapted for automatically performing the injection has come into practical use.

The above liquid injector typically has a liquid injection mechanism made up of, for example, a drive motor and a slider mechanism to allow a liquid syringe to be removably installed. The liquid syringe has a structure having a piston member slidably inserted into a cylinder member, and liquid syringes of a prefilled type and a refilled type have commonly been used.

A prefilled liquid syringe is filled with a predetermined liquid within the cylinder member and shipped in a sealed arrangement with packing materials. In the refilled liquid syringe, the user fills the cylinder member with a desired liquid. For simplification, explanation below is presented presuming that the liquid syringe is of the prefilled type.

When it is intended to inject the liquid in the foregoing liquid syringe into a patient, an operator accesses a pack of a liquid syringe for a proper liquid and takes the liquid syringe out of the pack. A sequence of operations of connecting the liquid syringe to the patient through an extension tube and deploying the liquid syringe in the liquid injection mechanism enables the liquid injector to cause a relative motion between the piston member and cylinder member through the operation of the liquid injection mechanism in response to a predetermined operation, thereby allowing the liquid to be injected from the liquid syringe to the patient.

In this case, the operator decides the rate of injection, the amount of the liquid to be injected, etc. taking a kind of the liquid etc. into account. Entering the decided result to the liquid injector as data enables the liquid injector to inject the liquid into the patient in accordance with the entered data. For example, suppose the case in which a contrast medium is injected as a liquid. Since the degree of contrast in a patient varies depending on the amount of the injected contrast medium, the diagnostic imaging apparatus provides a shoot of an excellent diagnostic image.

Further, in liquid injectors, there is a commercially available product capable of injecting a physiological saline as well as a contrast medium to a patient. When using such a product, the operator enters into the liquid injector as data, an injection of a physiological saline, if desired, to be linked to completion of the contrast-medium injection, in addition to the injection rate and amount.

In this case, the liquid injector injects the contrast medium and subsequently, automatically injects physiological saline to the patient as well in accordance with the entered data, thereby boosting up the contrast medium with the physiological saline to result in reducing the consumption of the contrast medium and also relieving an artifact by the physiological saline.

For reference, the above-described liquid injector has already been applied for patents by the present applicant etc. (for example, cf. Patent Documents 1 and 2.)
[Patent Document 1] JP 2002-11096
[Patent Document 2] JP 2002-102343

While the above-described liquid injector allows injecting liquid to a patient from a liquid syringe, it is required for an operator to select an appropriate liquid syringe in order to effect injection of a proper liquid. Since, however, a liquid syringe has the same appearance regardless of the kind of the liquid, the operator might deploy a liquid syringe for inappropriate liquid in a liquid injector.

Furthermore, a prefilled liquid syringe is thrown out to obviate infection when it has once used. However, a current liquid injector cannot prevent medical errors of reusing once-used liquid syringe.

Furthermore, it has been common practice to connect a liquid syringe to a patient through an extension tube and a needle-like member such as a catheter when using a liquid injector, and in this practice, the liquid injector injects the liquid at a higher pressure than the case of a manual operation. For this reason, the liquid injector requires employing pressure-withstanding liquid syringe and extension tube. However, it might be unavoidable that an inappropriate product will be employed for a syringe peripheral device such as an extension tube or catheter

Still further, while it is necessary to enter to the liquid injector as data, the injection rate and injection amount depending on the liquid to be used, such a work is complicated and difficult to the operators who are not skilled in the work. Consequently, it is unavoidable that inappropriate numerical values might possibly be entered. Particularly, existing contrast media include products that have active ingredients differing in concentration up to several times. Thus, ignoring the concentration difference in active ingredients will cause injection of a contrast medium ranging from one severalth to several times of an optimum quantity.

In like wise, a case may also occur in which it is required to enter data about an injection rate of liquid or the like to the liquid injector depending on the site to be imaged or weight of the patient. Such a work is also complicated and it is impossible to obviate an input error. The present applicant has applied for the patent in JP Application No. 2002-281109 claiming a liquid injector adapted for in particular varying an injection rate of a contrast medium to enhance an effect of the contrast medium. However, it is no easy task to establish the data of such a variable pattern in a liquid injector..

In order to solve the above-described problems, the present applicant has applied for the patent in JP Application No. 2003-098058 claiming a liquid injector adapted for reading the bar codes and the like indicating various items of data, which have been recorded on the packing material or the like of the liquid syringe. However, since a bar code has a small code capacity, it can represent only ID data and the like.

For this reason, in the above-described liquid injector, each of various items of data having a large amount of data such as a variable pattern is registered in advance, and the registered data are retrieved with read results from the bar codes. In order to do this, however, it is necessary to register various items of data into the liquid injector in advance and also, in such a case where the revision of the registered data is necessary, it is required that the liquid injector performs update of the data.

### SUMMARY OF THE INVENTION

The present invention has been made from the view of the above-described problems. It is an object of the present invention to provide a liquid injection system capable of facilitating an input of a large amount of data into a liquid injector to perform a variety of operations.

The liquid injection system according to the present invention is provided with a liquid syringe and a liquid injector. The liquid syringe has a cylinder member filled with a liquid and a piston member slidably inserted into the cylinder member, and is exchangeably deployed in the liquid injector. The liquid injector operates to move the cylinder member and piston member of the liquid syringe relatively to each other by means of the liquid injection mechanism to inject the liquid into the patient.

In this regard, the liquid syringe has two-dimensional codes representing predetermined data items, recoded thereon, and the liquid injector is provided with a code-reading means, a code-decoding means and an operation-controlling means. The code-reading means optically reads the two-dimensional codes, and the code-decoding means decodes the optically read two-dimensional codes. The operation-controlling means executes predetermined operations corresponding to the decoded results of the two-dimensional codes. Accordingly, recording, for example, a variable pattern for liquid injection etc. in terms of two-dimensional codes in the liquid syringe makes it possible for the liquid injector to inject the liquid in accordance with the predetermined variable pattern, thereby allowing an easy input of a great amount of data to the liquid injector and enabling execution of various kinds of operations.

For reference, it suffices each means disclosed in the present invention that the means is formed to be capable of satisfactorily realizing the function: for example, the means can be realized as hardware dedicated to realizing a predetermined function, a data processor device having a predetermined function provided in accordance with a computer program, a predetermined function realized by a data processor device in accordance with a computer program, and a combination of these means.

Further, each of the various constituent elements disclosed in the present invention is not necessarily an element individually independent of other elements, and can be a single member made up of a plurality of constituent elements. Alternatively, a case is also possible such that one constituent element is a part of another constituent element, that one constituent element and another constituent element are partly overlapped, or the like.

In addition, the two-dimensional code described in the present invention refers to the coded data in the two-dimensional code format that are optically readable by a CCD camera and decodable by a computer device etc., specifically the QR code, the data matrix, the PDF 417, the maxicode, the varicode, etc. being available.

### BRIEF DESCRIPTION OF THE DRAWINGS

Fig. 1 is a schematic block diagram representing a logic structure of the liquid injector of an embodiment according to the present invention;
Fig. 2 is a perspective view representing a manner of mounting syringes on a injection head of the liquid injector;
Fig. 3 is a perspective view representing an exterior appearance of the liquid injector;
Fig. 4 is a perspective view representing an exterior appearance of an MRI apparatus that is a diagnostic imaging apparatus;

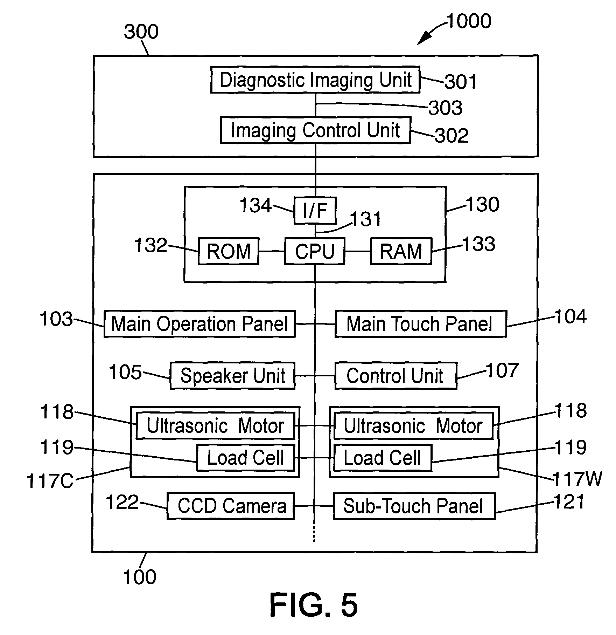

Fig. 5 is a block diagram illustrating the circuitry of the liquid injector;
Fig. 6 is a flow chart representing a former half of the processing operations of the liquid injector;
Fig. 7 is a flow chart representing a latter half; and
Fig. 8 is a flow chart representing the processing operations of the MRI apparatus.

### DETAILED DESCRIPTION OF THE PREFERRED EMBODIMENTS

### [Configuration of the embodiment]

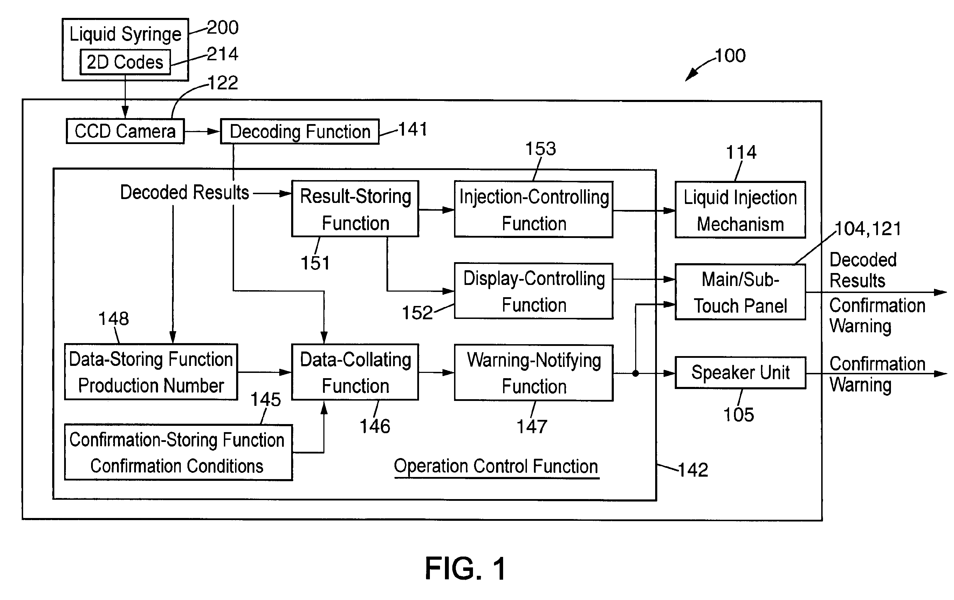

Explanation is presented below regarding an embodiment of the present invention referring to drawings. The liquid injection system 1000 of an embodiment according to the present invention, comprises liquid injector 100, liquid syringe 200 and MRI apparatus 300, which is a diagnostic imaging apparatus, as illustrated in Fig. 1 through Fig. 4. The system is intended for injecting a contrast medium or the like as a liquid to a patient (not shown) as will be described in detail later.

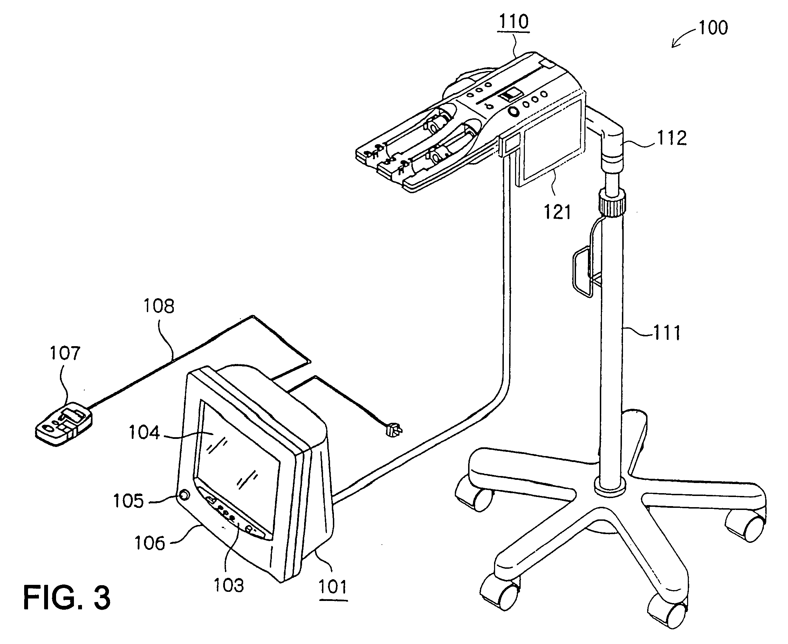

The MRI apparatus 300 is provided with diagnostic imaging unit 301, which is an installation for implementing imaging, and imaging control unit 302, as shown in Fig. 3, with diagnostic imaging unit 301 and imaging control unit 302 wired-connected through communication network 303. Diagnostic imaging unit 301 shoots a diagnostic image of a patient, and imaging control unit 302 controls the operation of diagnostic imaging unit 301.

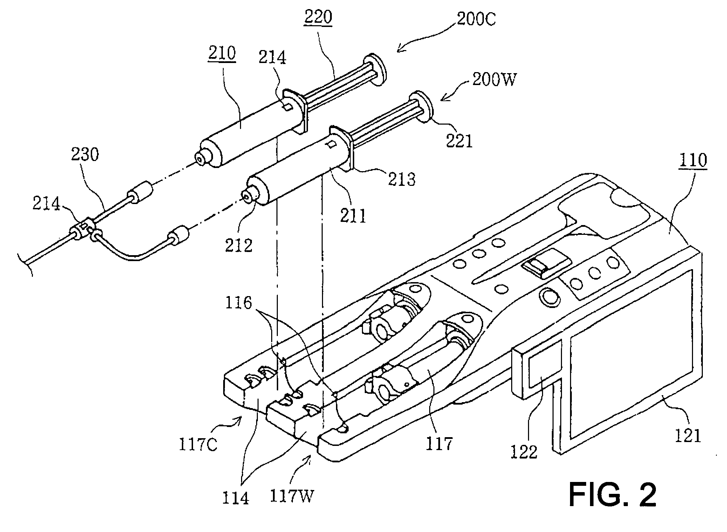

Liquid syringe 200 comprises cylinder member 210 and piston member 220, wherein piston member 220 is slidably inserted into cylinder member 210, as illustrated in Fig. 2. Cylinder member 210 is provided with cylindrical hollow body 211, which has conduit tube 212 formed in the closed leading end surface.

The trailing end of body 211 of cylinder member 210 is opened and piston member 220 is inserted from the opening into the interior of body 211. Cylinder member 210 has cylinder flange 213 formed in the outer circumference of the trailing end, and piston member 220 has piston flange 221 formed in the outer circumference of the trailing end.

At least a part of the employed liquid syringes 200 is of the prefilled type in liquid injection system 1000 of the present embodiment, and liquid syringe 200 of the prefilled type is shipped with cylinder member 210 being filled with liquid.

Cylinder member 210 of liquid syringe 200 has 2D (two-dimensional) codes 214 printed or labeled on the outer circumference thereof, wherein 2D codes 214 represent a variety of items of the data with regard to liquid syringe 200 of interest such as the name; the ID data to identify the prefilled type or refilled type; the ID data for identifying each syringe piece; the capacity; the withstand pressure of cylinder member 210; the inner diameter of cylinder member 210; the stroke of piston member 220, etc.; established in the 2D code.

Further, if liquid syringe 200 is of the prefilled type, then further variety of data items about the filled liquid, such as the name, ingredients, viscosity, expiration date, and ID data indicating whether the intended use is for CT or for MR, are established and coded to be 2D codes 214. Furthermore, if the liquid filled in prefilled liquid syringe 200 is a contrast medium, then further data items such as a variable pattern of varying an injection rate with passage of time are established as required and coded in the 2D code to form part of 2D codes 214.

For reference, liquid syringe 200 includes contrast syringe 200C filled with a contrast medium as the liquid and also food syringe 200 W filled with physiological saline as the liquid. Contrast and/or food syringes 200C, 200W can be installed in liquid injector at the same time.

Contrast and/or food syringes 200C, 200W installed in liquid injector 100, as described above, are connected to a patient through a syringe peripheral device, for example, two-forked extension tube 230, wherein 2D code 214 is also assigned to such a syringe peripheral device and a variety of items of data such as the name and withstand pressure of the syringe peripheral device are established and coded in the 2D code to constitute 2D codes 214.

As shown in Fig. 3, liquid injector 100 of the present embodiment 100 has injection control unit 101 and injection head 110 constructed as separate units, which are wired-connected through communication cable 102.

Injection head 110 drives installed liquid syringe 200 to inject a liquid to a patient, and injection control unit 101 controls the operation of injection head 110. For this end, computer unit 130 is built in injection control unit 101 as shown in Fig. 2, and injection control unit 101 is wired-connected to imaging control unit 302 of MRI apparatus 300 through communication network 304.

Injection control unit 101 has main operation panel 103, main touch panel 104, which is a means for displaying data, speaker unit 105, etc. arranged on the front face of main body housing 116 and is wired-connected via joining connector 108 to control unit 107, which is a separate unit.

Injection head 110 is attached to the top end of caster stand 111 with movable arm 112, and as shown in Fig. 2, head body 113 has concave portions 114 formed in semi cylindrical grooves on the upper surface to adapt for removably attaching liquid syringe 200. Concave portion 114 has cylinder-holding mechanism 116 for removably holding cylinder flange 213 of liquid syringe 200 formed in the forward section and also has liquid injection mechanism 117 for holding and slidingly moving piston flange 221 arranged in the rearward section.

Cylinder-holding mechanism 116 is formed in concave portions 114 in a form of an anomalous reentrant groove, with which each of cylinder flanges 213 removably engages. Liquid injection mechanisms 117 individually have ultrasonic motors 118, which are free from generation of magnetic field even when it is operated, as power sources and slidingly move piston members 220 through screw mechanisms or the like (not shown). Further, liquid injection mechanisms 117 individually have built-in load cells 119, which detect pressures applied to piston members 220.

Because two concave portions 114 of Injection head 110 are respectively adapted for receiving contrast and/or food syringes 200C, 200W, these two concave portions 114 and two liquid injection mechanisms 117 constitute both contrast-medium injection mechanism 117C for injecting a contrast medium and food injection mechanism 117W for injecting physiological saline, to a patient.

In addition, in liquid injector 100 of the present embodiment at least respective elements of injection head 110 are formed of nonmagnetic material, and the portions that cannot be formed of nonmagnetic material are magnetically shielded.

For example, ultrasonic motor 118, load cell 119, etc. are formed of nonmagnetic metals such as phosphor bronze alloy (Cu + Sn +P), titanium alloy (Ti-6Al-4V) and magnesium alloy (Mg + Al +Zn) and head body 113 and the like are formed of nonmagnetic resin.

Injection head 110 has sub-touch panel 121 that serves as a data displaying means and CCD (Charge Coupled Device) camera 122 that serves as a code reading means, both arranged on a side surface of the rear portion, and CCD camera 122 optically reading CD codes 214 of liquid syringe 200, extension tube 230, etc.

In liquid injector 100 of the present embodiment, the above-described variety of devices is connected to computer unit 130 as shown in Fig. 2, which performs comprehensive controls of the variety of devices. Computer unit 130 is made of a so-called one-chip microcomputer provided with hardware such as a CPU (Central Processing Unit), ROM (Read Only Memory) 132, RAM (Random Access Memory) 133, an I/F (Interface) 134, etc.

Computer unit 130 has an appropriate computer program installed in an information storage medium such as ROM 132 as firmware or the like, and CPU 131 executes various processes in accordance with the computer program.

Since computer unit 130 operates in accordance with the computer program installed as described above, liquid injector 100 of the present embodiment logically has a variety of functions shown in Fig. 1 such as code-decoding function 141, operation control function 142, etc. as corresponding variety of means.

Code-decoding function 141 corresponds to the function of CPU 131 to execute predetermined processes in accordance with the computer program loaded in ROM 132, or the like, and decodes 2D codes 214 optically read by CCD camera 122, .

Operation control function 142 corresponds to the function of CPU 131 to execute predetermined operations corresponding to the computer program and the decoded results of 2D codes 214, and comprises confirmation-storing function 145, data-collating function 146, warning-notifying function 147, data-storing function 148, result-storing function 151, display-controlling function 152 and injection-controlling function 153.

Confirmation-storing function 145 corresponds to the storage area allotted to RAM 133 to allow CPU 131 to confirm data and stores predetermined confirmation conditions as data. Data-collating function 146 acts to collate the confirmation conditions stored as data and the decoded results of 2D codes 214. Warning-notifying function 147 provides an output of notifying a confirmation warning in accordance with the collation result.

More specifically, the identification data of usable liquid syringes 200, extension tubes, etc. are saved in RAM 133 as confirmation conditions. When CCD camera 122 optically reads 2D codes 214 of liquid syringe 200 and extension tube 230 and the read codes are decoded by CPU 131, the decoded identification data of liquid syringe 200, extension tube 230, etc. are collated with the identification data saved in RAM 233.

If the decoded identification data are not saved, the guidance message such as "this product is not registered as a usable device; please confirm whether or not it is usable" is displayed on main/sub-touch panel 104, 121 as a confirmation warning and also provided as a voice output through speaker unit 105.

Furthermore, RAM 133 stores daily updated current date and hour as a confirmation condition, and when the expiration date for a safe use is decoded from 2D codes 214 of liquid syringe 200, the expiration date is collated with the current date and hour. If the current date runs over the expiration date, the guidance message such as "this product runs over the expiration date; please employ a new product" is displayed on main/sub-touch panel 104, 121 as a confirmation warning and also provided as a voice output through speaker unit 105.

Further, since prefilled liquid syringe 200 has a production number established for every product and coded in the 2D code to constitute 2D codes 214, data-storing function 148 stores the production number of prefilled liquid syringe 200 that has been installed in injection head 110 and has experienced a performance of the injection operation.

In this case, data-collating function 146 collates the stored production number and the production number decoded from 2D code 214, and if the collated production numbers coincide, then warning-notifying function 147 operates to display the guidance message such as "this product has already been used in the past; please employ a new product" on main/sub-touch panel 104, 121 as a confirmation warning and also provide the warning as a voice output through speaker unit 105.

Result-storing function 151 operates to store the decoded result of 2D codes 214, display-controlling function 152 operates to display the stored decoded results on main/sub-touch panel 104, 121, and injection-controlling function 153 operates to control the operation of liquid injection mechanism 117 in accordance with the stored decoded results.

More specifically, in 2D codes 214 of liquid syringe 200 are established various items of data about liquid syringe of interest 200 such as the name, withstand pressure and volume and also about the liquid filled in liquid syringe of interest 200 such as the name, ingredients and expiration date for safe use, and these various items of data are temporarily stored in RAM 133 and then supplied to display on main/sub-touch panel 104, 121.

Further, if the control data of liquid injection mechanism 117 have been established in 2D codes 214 of liquid syringe 200, the control data are stored in RAM 133 and CPU 131 controls the operation of liquid injection mechanism 117 in accordance with the stored control data. For example, if 2D codes 214 of contrast syringe 200 involve a variable pattern established to vary an injection rate of the contrast medium with time, CPU 131 varies the operation speed of liquid injection mechanism 117 for a contrast medium with a time passage in accordance with the variable pattern.

Furthermore, if 2D codes of liquid syringe 200 and extension tube 230 are assigned to the data about their withstand pressures, CPU 131 controls the operation of liquid injection mechanism 117 not to exceed the withstand pressures stored in RAM 133 depending on the pressure detected by load cell 119.

If 2D code 214 of liquid syringe 200 is assigned to the data about a volume, CPU 131 controls the operation of liquid injection mechanism 117 depending on the volume stored in RAM 133. In addition, when 2D codes 214 of contrast syringe 200C and food syringe 200W are sequentially read, CPU 131 operates sequentially contrast-medium injection mechanism 117C and food injection mechanism 117W.

While a variety of functions of liquid injector 100 can be realized utilizing hard ware such as main/sub-touch panel 104, 121 as occasion demands, the essential part can be realized by the resources stored in the information storage media such as ROM 132 etc. and the functioning of CPU 131, which is hardware, in accordance with a computer program.

Such a computer program is stored in the information storage media such as RAM 133 etc. as software for activating CPU 131 etc., to execute processes of: decoding 2D codes 214 optically read through CCD camera 122; collating the confirmation conditions stored in RAM 133 etc. and the decoded results of 2D code 214; notifying confirmation warnings in accordance with the collation results through a display device etc. such as main/sub-touch panel 104, 121; storing the production numbers of liquid syringes 200 that have been installed and experienced the performance of the injection operations; collating the stored production number and the production number decoded from 2D codes 214; notifying confirmation warnings in accordance with the collation results through a display device etc. and the like such as main/sub-touch panel 104, 121; storing the decoded results of 2D codes 214 in RAM 133 etc.; displaying the stored decoded results on main/sub-touch panel 104, 121; and controlling the operations of liquid injection mechanism 117 corresponding to the stored decoded results.

### [Operation of the Embodiment]

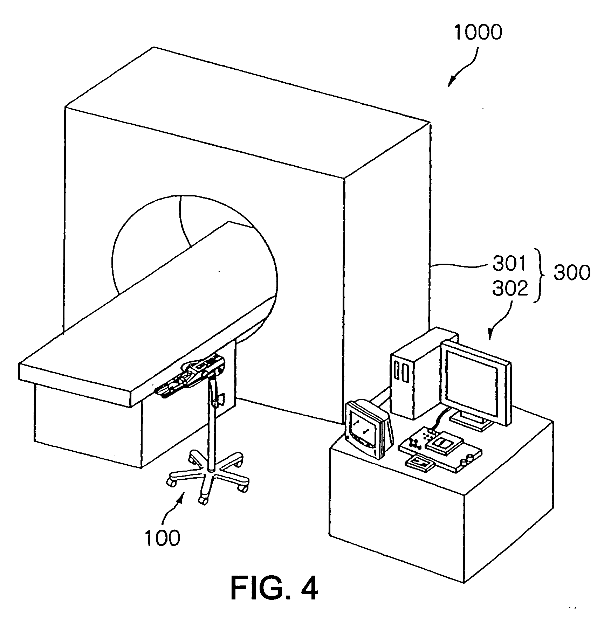

When using liquid injector 100 of the present embodiment in the above-described configuration, an operator (not shown) arranges liquid injector 100 near imaging unit 301 of MRI apparatus 300 as shown in Fig. 4 and prepares contrast and/or food syringe 200C, 200W and extension tube 230.

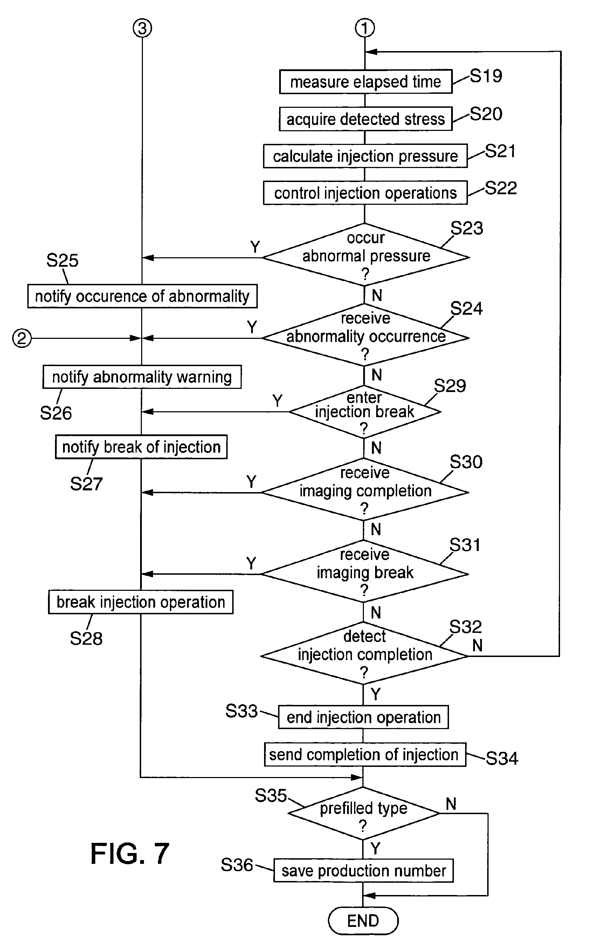

Next, 2D codes 214 of contrast and/or food syringe 200C, 200W and extension tube 230 are placed in opposed positions to CCD camera 122 of injection head 110 in liquid injector 100, and this CCD camera 122 optically reads 2D codes 214 as shown in Fig. 6 (Step S1).

Then, the optically read 2D codes 214 are decoded by computer unit 130 (Step S2), and the decoded data are collated with the confirmation conditions saved in RAM 133 (Step S3). The identification data of available liquid syringes 200, extension tubes 230, etc. are saved as confirmation conditions in this case. Accordingly, if the identification data of liquid syringe 200 etc. decoded from 2D codes 214 are not saved as the confirmation conditions, a guidance message such as "this product is not registered as an available device; please confirm whether or not it is available" is displayed on main/sub-touch panel 104, 121 as a confirmation warning, and also provided as a voice output through speaker unit 105 (Step S4).

In this occasion, since a guidance message such as "do you want to register this product as an available product? Y/N" is also displayed on main/sub-touch panel 104, 121 and further given as a voice output through speaker unit 105, an input operation to enter the expression "Y" onto main/sub-touch panel 104, 121 (Step S5) provides the registration of the identification data in RAM 133 as the confirmation conditions (Step S6).

Alternatively, if an input operation to enter the expression "N" onto main/sub-touch panel 104, 121 (Step S5) is made (Step S5), liquid injector 100 recovers its initial state. As a result, the operator prepares a new proper product and starts the operation again (Steps S1 through S3).

Furthermore, if the expiration date for a safety use decoded from 2D codes 214 of liquid syringe 200 runs over the current date and hour (Step S3), the guidance message such as "this product runs over the expiration date; please employ a new product" is notified on main/sub-touch panel 104, 121 and through speaker unit 105 as a confirmation warning (Step S4), because the current date and hour are also saved as a confirmation condition.

It should be appreciated that because storing again a new confirmation condition is not performed in this case of running over the expiration date, and liquid injector 100 automatically restores its initial condition (Step S5), the operator will prepare new liquid syringe 200 that has not pass the expiration date yet and restart the process (Steps S1 through S3).

When the above-described check on the conformance to the confirmation conditions has been completed, it is decided from the decoded results of the 2D codes 214 whether or not the product of interest is liquid syringe 200 of a prefilled type (Step S7). If the liquid syringe 200 is of the prefilled type, then the production number decoded from 2D codes 214 is collated with the production number saved in RAM 133 (Step S 8).

If the collated production numbers are coincident with each other, then a guidance message such as "this product has already been used in the past; please employ a new product" is notified on main/sub-touch panel 104, 121 and through speaker unit 105 as a confirmation warning (Step S9).

In this case also, liquid injector 100 restores its initial state, and consequently the operator prepares a new prefilled liquid syringe 200 that has not employed yet and restarts the process (Steps S1 through S3).

If the product is not prefilled liquid syringe 200 (Step S7), or if the product number of prefilled liquid syringe 200 is not saved (Step S8), the decoded result of 2D codes 214 is stored in RAM 133 and displayed on main/sub-touch panel 104, 121 (Step S10).

More specifically, because 2D codes 214 of liquid syringe 200 are assigned to various kinds of the data items about liquid syringe 200 such as the name, withstand pressure, and volume and also various kinds of the data items about the liquid filled in the liquid syringe of interest such as the name, ingredients, expiration date for a safe use, etc., these various kinds of the data items are temporary stored in RAM 133 to display on main/sub-touch panel 104, 121.

For reference, because 2D codes 214 are assigned to various kinds of both data items intended for display and not intended for display, a binary flag, for example, is assigned to each of the various kinds of data items to indicate whether or not the item of interest is intended for display. Liquid injector 100 displays appropriate members of the items based on the decoded results of 2D codes 214.

Next, the control data for controlling liquid injection mechanism 117 are extracted from the decoded results of 2D codes 214, which are installed into RAM 133 (Step S 11). For reference, if established control data are not included in the decoded results of 2D codes 214, the default control data are installed into RAM 133.

When the operator places 2D codes 214 of contrast and/or food syringe 200C, 200W and extension tube 230 in opposed positions to CCD camera 122 of injection head 110 in liquid injector 100, the confirmation warnings and the decoded results of 2D codes are displayed on sub-touch panel 121 of injection head 110 and the control data for controlling liquid injection mechanism 117 are established.

Next, the operator connects contrast and/or food syringe 200C, 200W to a patient (not shown) located in imaging unit 301 and deploys cylinder member 210 of the liquid syringe 200 into injection head 110.

When the operator makes an input operation to command main/sub-touch panel 104, 121 and main operation panel 103 to start operations, liquid injector 100 detects the input operation (Step S 12) and sends the signal indicating the start of operation to MRI apparatus 300 (Step 15).

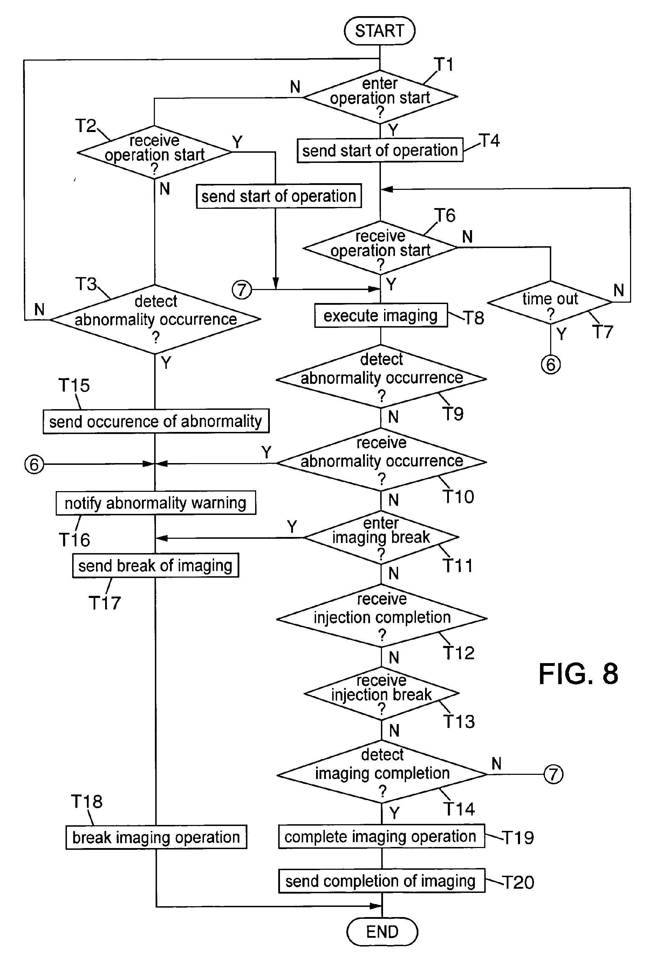

Now referring to Fig. 8, when MRI apparatus 300 receives the signal indicating the operation start from liquid injector 100 as described above (Step T2), MRI apparatus 300 returns the signal notifying the operation start to liquid injector 100 and executes an operation of diagnostic imaging (Step T8). For this end, the imaging operation of MRI apparatus 300 follows up the liquid injection made by liquid injector 100 in the diagnostic imaging system 1000 of this embodiment.

For reference, in diagnostic imaging system 1000 of the present embodiment, when liquid injector 100 is in ready state as described above (Steps S12 through S 14) and an input operation is made to command MRI apparatus 300 to start an imaging operation (Step T1) as represented in Fig. 6 and Fig. 8, liquid injection of liquid injector 100 follows up the diagnostic imaging of MRI apparatus 300 (Steps T4, from T6 onward, S 13, from S18 onward).

In liquid injector 100, when executing a sequence of operations to inject the liquid (from Step S19 onward), the elapsed time is measured from the start of injection (Step S19) and the operations of contrast-medium injection mechanism 117C and food injection mechanism 117W are sequentially controlled in real time corresponding to the above-described elapse time and the control data decoded from 2D codes 214 (Step S22), as represented in Fig. 7.

For this end, if a variable pattern is established in 2D codes 214 of contrast syringe 200C to vary the injection rate of the contrast medium with passage of time, the operation speed of contrast-medium injection mechanism 117C is varied with time in accordance with the variable pattern.

Furthermore, when liquid injection mechanism 117 is driven as described above, computer unit 130 acquires the stress detected by load cell 142 in real time (Step S20).

Then, the injection pressure of the liquid is calculated from the stress detected by load cell 142 corresponding to the viscosity of the liquid and the inner diameter of cylinder member 210 decoded from 2D codes 214 (Step S21), and the operation of liquid injection mechanism 117 is controlled in real time so that the injection pressure will not exceed the pressure range decoded from 2D codes 214 (Step S23).

Further, in liquid injector 100 and MRI apparatus 300 of the present embodiment, if an occurrence of abnormality is detected in ready state described above (Steps S14, T3), or if an occurrence of abnormality is detected in the course of making an operation (Steps S23, T9), the occurrence of abnormality is notified (Steps S26, T 16) and also a break of the operation is executed (Steps S28, T18).

Since the occurrence of abnormality is notified also to another apparatus (Steps S25, T15), the other apparatus that receives the notification of the occurrence of abnormality also provides a notification of the occurrence of abnormality (Steps T16, S26). Further, since a break of an operation in one apparatus is also notified to another apparatus (Steps S27, T17), the other apparatus that receives the notification of the break of the operation (Steps T13, S31) also executes a break of an operation (Steps T18, S28).

Furthermore, when an input operation is made in one apparatus to command to break an operation (Steps S29, T11), the beak of an operation is executed in the apparatus of interest (Steps S28, T18) and also notified to another apparatus (Steps S27, T17). As a result, the beak of an operation is executed (Steps T18, S28) in the other apparatus that receives the notification (Steps T13, S31).

Furthermore, when the completion of the operation is detected in one apparatus (Steps S32, T14), the completion of the operation is executed in the apparatus of interest (Steps S33, T19) and also notified to another apparatus (Steps S34, T20). As a result, the completion of the operation is executed (Steps T18, S28) in the other apparatus that receives the notification (Steps T12, S31)

In liquid injector 100 of the present embodiment, if liquid syringe 200 is of the prefilled type (Step S35), the identification data decoded from 2D codes 214 of the liquid syringe 200 of interest are saved in RAM 133 as a confirmation condition (Step S36) when the injection operation is normally or abnormally completed (Steps S33, S28).

### [Effect of the Embodiment]

In liquid injection system of the present embodiment 1000, a sequence of the operations of recording a variety of items of data in the 2D codes 214 on liquid syringe 200, optically reading 2D codes 214, decoding them, and executing predetermined operations by liquid injector 100 makes it possible to enter a great amount of data to liquid injector 100 and execute a variety of operations.

Particularly, because 2D code 214 has a large code capacity as compared with a bar code, it is possible to save a variety of items of data together with their identification codes in liquid injector 100 in advance, there is no need to retrieve the saved data using the identification code decoded from a bar code, and even a great amount of completely new data can easily be entered to liquid injector 100.

Furthermore, in liquid injection system 1000 of the present embodiment, it is possible for an operator to confirm simply and reliably a variety of items of data about liquid syringe 200 etc. to be used, because at least a part of the decoded results of 2D codes 214 is stored and displayed on main/sub-touch panel 104, 121.

Particularly, the arrangement of mounting CCD camera 122 and sub-touch panel 121 on injection head 110 having liquid syringe 200 installed therein allows displaying a variety of items of decoded data on sub-touch panel 121 adjoining CCD camera 122 when the operator places 2D code 214 of liquid syringe 200 installed in injection head 110 in a position opposing CCD camera 122, whereby it is enabled to confirm simply and intuitively the variety of items of data about liquid syringe 200 etc. to be employed.

Furthermore, because sub-touch panel 121 for displaying the decoded results of 2D codes 214 is capable of receiving an input operation as well, the operator can adjust various operations of liquid injector 100 with ease as desired when liquid injector 100 performs the various operations in accordance with the decoded results of 2D codes 214.

In addition, liquid injector 100 of the present embodiment is capable of collating the saved confirmation conditions and a decoded result of 2D code 214 and notifying a confirmation warning as occasion demands. In this way, for example, if someone intends to employ liquid syringe 200 unusable for liquid injector 100 of interest, or to employ liquid syringe 200 that has passed an expiration date for safe use, the confirmation warning is notified, thereby satisfactorily preventing various medical errors.

Particularly, in liquid injector 100 of the present embodiment, when 2D codes 214 of prefilled liquid syringes 200 are read, a production number of every syringe member is stored, and if a production number newly decoded from a 2D code has alredy been stored, a confirmation warning is notified, thereby obviating medical errors such that a prefilled liquid syringe 200, which is to be thrown away if it has been once used, is repeatedly used.

Further, it is enabled to employ prefilled and refilled liquid syringes 200 purposively, because liquid injector 100 of the present embodiment detects whether liquid syringe 200 of interest is of a prefilled-type or a refilled-type from the decoded result of 2D code 214 and the above-described operations are performed only in the case of the prefilled type.

Furthermore, in liquid injection system 1000 of the present embodiment, in the case that a variable pattern, which indicates a time-dependent variation of the injection rate of a contrast medium, is established in 2D codes 214 of a prefilled liquid syringe 200 for a contrast medium, liquid injector 100 varies the injection rate of the contrast medium with passage of time in accordance with the variable pattern.

Consequently, it is enabled to properly maintain an optimum degree of contrast, whereby the injection volume of the contrast medium is kept minimum necessary, thereby reducing a physical burden on a patient. Nevertheless, it is feasible to enter easily, for example, even a new variable pattern corresponding to a new contrast medium to liquid injector 100 from 2D codes 214 of liquid syringe 200 without necessitating any registration of a complex variable pattern in liquid injector 100 in advance.

Furthermore, liquid injector 100 of the present embodiment can prevent a medical error of injecting a liquid under an abnormal pressure, because the injection pressure of the liquid is detected from the stress that presses piston member 220 of liquid syringe 200 and, when the injection pressure becomes an abnormal pressure, an confirmation warning is issued and also the injection operation is forcedly halted.

For reference, in order that liquid injector 100 detects the pressure of the liquid as described above, not only the stress that presses piston member 220 of liquid syringe 200 but also a variety of items of data such as the inner diameter of cylinder member 210, the viscosity of the liquid, etc. are required. Such a variety of items of data, however, are entered to liquid injector 100 as 2D codes 214.

Thus, liquid injection system 1000 of the present embodiment allows liquid injector 100 to detect purposively the injection pressure for every liquid syringe and also for every liquid without necessitating complicated operations of the operator to manually enter various items of data to liquid injector 100.

Furthermore, in liquid injection system 1000 of the present embodiment, 2D codes 214 assigned to not only liquid syringe 200 but also the syringe peripheral devices such as extension tube 230 etc are recorded. Consequently, it is enabled for liquid injector 100 to control the injection operation adaptive to a withstand pressure of extension tube 230 etc., thereby properly obviating a medical error such that extension tube 230 unusable for liquid injector of interest 100 is employed.

Still further, in liquid injection system 1000 of the present embodiment, since the liquid injection performed by liquid injector 100 and the shooting of images made by MRI apparatus 300 are automatically linked to each other, it is enabled to shoot in appropriate timings diagnostic images from the patient, who has had sequential injection treatments of a contrast medium and a physiological saline in proper timings,

### [Variation of the Embodiment]

The present invention is not limited to the above-described embodiment and permits wide variations within a scope not departing from the gist of the invention. For example, it is exemplified in the above-described embodiment that satisfactory performances of operations are created by such an arrangement that CCD camera 122 for optically read 2D codes 214 and sub-touch panel 121 for displaying the decoded results are arranged in injection head 110 in which liquid syringe 200 is deployed.

It is also possible, however, to deploy CCD camera 122 and sub-touch panel 121 in the positions separated from injection head 110, and it is further possible to connect CCD camera 122 as an isolated handy unit to liquid injector 100 by a wired or wireless connection (not shown).

Further, because digital cameras capable of optically reading 2D codes and portable telephones in which such digital cameras are incorporated have been in widespread use, it is also possible to connect such a device as a means for reading codes to liquid injector 100 by a wired or wireless connection (not shown).

In addition, while the above-described embodiment exemplifies an arrangement in which CCD camera 122 is located on one side of injection head 110, it is also feasible to deploy CCD camera 122 in the position (not shown) where the optical reading is performed of 2D codes 214 of liquid syringe 200 installed in the liquid injector. In this case, because 2D codes 214 are automatically read optically by CCD camera 122 when liquid syringe 200 is installed in injection head 110, the operation of the operator to direct 2D codes 214 toward CCD camera 122 becomes unnecessary.

Furthermore, a liquid injector having the above-described structure can be configured to permit liquid injection mechanism 117 to operate only when CCD camera 122 optically detects 2D code 214. In this case, because liquid injection mechanism 117 operates only when liquid syringe 200 is properly implemented in injection head 110, it is feasible to automatically halt liquid injection mechanism 117 when, for example, liquid syringe 200 drops out of injection head 110.

Furthermore, while the above-described embodiment exemplifies the recording of 2D codes 214 on the outer circumferential surface of cylinder member 210 of liquid syringe 200, it is also feasible, for example, to record 2D codes 214 on the outer side surface or trailing end surface, or alternatively to record on a packing material of liquid syringe 200 (not shown).

Furthermore, while the above-described embodiment exemplifies recording of 2D codes 214 on liquid syringe 200 and extension tube 230, it is also feasible to record 2D codes 214, for example, only on liquid syringe 200, or alternatively to record 2D codes 214 on each of various kinds of syringe peripheral devices other than extension tube 230, such as a catheter or a liquid bottle (not shown).

Furthermore, while the above-described embodiment exemplifies recording of 2D codes 214 on liquid syringe 200 and extension tube 230, it is also feasible to record 2D codes 214, assigned to various kinds of data items about a patient, on an accessory of a patient, for example, a wrist band arranged around a wrist of the patient, a medical card on which various items of data about the patient are listed, etc. (not shown).

In this case, because various data items about the patient can simply be entered into liquid injector 100, it becomes feasible, for example, to control the injection operation corresponding to the weight and age of the patient, thereby automatically obviating injection of the liquid irrelevant to the patient's disease.

Furthermore, while the above-described embodiment exemplifies entering various data items of liquid syringe 200 etc. into liquid injector 100 in terms of 2D codes 214, it is also feasible, for example, to update the data of a computer program and the data of resources of liquid injector 100 using such 2D codes 214 of the entered data items.

Furthermore, while the above-described embodiment exemplifies injection of both a contrast medium and physiological saline through liquid injector 100 provided with contrast and/or saline injection mechanism 117C, W, it is feasible to embody a liquid injector for injecting only a contrast medium through the use of single liquid injection mechanism 117, or to embody a liquid injector for injecting more than two kinds of liquid through the use of more than two liquid injection mechanisms 117 (neither shown).

Further, while the above-described embodiment exemplifies employing MRI apparatus 300 as a diagnostic imaging apparatus and injecting a contrast medium for MR using liquid injector 100, it is possible, for example, to employ a CT scanner or a PET apparatus as a diagnostic imaging apparatus and inject a contrast medium adapted for the employed apparatus through the liquid injector.

Further, while the above-described embodiment exemplifies a configuration in which various kinds of functions of liquid injector 100 are logically realized through the operation of CPU 131 in accordance with a computer program stored in RAM 133 etc., it is also possible, however, to realize each of such various functions as the function-specific hardware and it is alternatively possible to store a part of such functions as software in RAM 133 and embody other part of the functions as hardware.

Further, while it is presumed in the above-described embodiment that manufacturers record 2D codes 214 on a liquid syringe 200 and an extension tube 230, it is also feasible, for example, to print 2D codes 214 on stickers on the site of a hospital etc. where the liquid syringes 200 etc. are used and to stick the stickers on liquid syringes 200 etc. to provide 2D codes to liquid syringes 200 etc.

In this case, it is enabled, for example, to record various data items about liquids on refilled liquid syringes 200 in terms of 2D codes 214, because it is feasible to provide desired data on site to liquid syringe 200. Furthermore, the data of 2D codes 214 to be printed and used on site can, for example, be supplied from the manufacturer to the site as an attached data of an email, or can be provided on a home page of the manufacturer as predetermined down-loadable data.

Further, while the above-described embodiment exemplifies an arrangement in which CCD camera 122 for optically reading 2D codes 214 of liquid syringe 200 is installed in liquid injector 100, it is also feasible, for example, to mount CCD camera 122 in a liquid-temperature retaining apparatus for keeping liquid syringe 200 at an appropriate temperature (not shown).

In this case, it is possible that the liquid-temperature retaining apparatus keeps the liquid at an appropriate temperature by controlling the warm-keeping operation corresponding to the decoded result of 2D codes 214 and also it is possible that the liquid-temperature retaining apparatus transmits the read results and decoded results of 2D codes 214 to a liquid injector in which CCD camera 122 is not mounted.

## Claims

1. A liquid injection system 1000 including at least a liquid syringe 200 having a piston member 220 slidably inserted into a cylinder member 210 filled with a liquid, and also a liquid injector 100 that moves said cylinder member 210 and said piston member 220 of the exchangeably mounted liquid syringe 200 relatively to each other through a liquid injection mechanism 117 to inject said liquid into a patient; wherein
said liquid syringe 200 has various kinds of data items recorded in the two-dimensional code 214, and
said liquid injector 100 comprises:
a code-reading means 122 for optically reading two-dimensional codes 214,
a code-decoding means 141 for decoding the optically read two-dimensional codes 214, and
an operation-controlling means 142 for executing predetermined operations corresponding to the decoded results of said two-dimensional codes 214.

2. A liquid injection system according to claim 1, wherein said liquid injector has a data-displaying means for displaying various kinds of data items, and
said operation-controlling means has a result-storing means for storing the decoded results of said two-dimensional codes and a display-controlling means for displaying at least a part of the stored decoded results on said data-displaying means.

3. A liquid injection system according to claim 2, wherein said liquid injector includes an injection control unit in which at least said operation-controlling means is mounted; and an injection head formed separately from said injection control unit and having at least said liquid injection mechanism and said data-displaying means mounted therein.

4. A liquid injection system according to claim 3, wherein said code-reading means is also mounted in said injection head.

5. A liquid injection system according to claim 1, wherein said liquid injector has said code-reading means arranged in the position where said two-dimensional codes of the mounted liquid syringe are optically read.

6. A liquid injection system according to claim 5, wherein said operation-controlling means enables said liquid injection mechanism to operate only when said code-reading means optically detects said two-dimensional codes.

7. A liquid injection system according to any one of claims 1 through 6, wherein said operation-controlling means has a result-storing means for storing the decoded results of said two-dimensional codes and an injection control means for controlling the operations of said liquid injection mechanism corresponding to at least a part of the stored decoded results.

8. A liquid injection system according to claim 7, provided with a prefilled liquid syringe that is shipped with a contrast medium being filled as a liquid to be injected to a patient when a diagnostic image of the patient is shot through a diagnostic imaging apparatus, wherein
a variable pattern for varying the injection rate of said contrast medium with time is established in the two-dimensional codes of said liquid syringe, and
said operation-controlling means varies the speed of operation of said liquid injection mechanism with time in accordance with said variable pattern.

9. A liquid injection system according to any one of claims 1 through 6, wherein said operation-controlling means is provided with a confirmation storing means for storing predetermined confirmation conditions, a data collating means for collating the stored confirmation conditions and the decoded results of said two-dimensional codes, and a notifying means for notifying a confirmation warning corresponding to the collated results.

10. A liquid injection system according to any one of claims 1 through 6, provided with a prefilled liquid syringe that is shipped with a liquid being filled, said prefilled liquid syringe having at least a production number for every syringe established in said two-dimensional code, wherein
said operation-controlling means is provided with data storing means for storing said production number of said liquid syringe that has been deployed and activated to execute an injection operation, a data collating means for collating said production number that has bee stored and said production number that has been newly read, and a warning means for notifying a confirmation warning when the collated production numbers coincide with each other.

11. A liquid injection system according to any one of claims 1 through 6, further provided with syringe peripheral devices including a hollow needle-like member adapted for inserting into a patient and flowing said liquid, an extension tube for connecting said needle-like member and said liquid syringe to flow said liquid, and a one-way valve inserted in said extension tube for controlling the flow direction of said liquid, wherein
the two-dimensional codes representing various kinds of data items established in connection to each of said syringe peripheral devices are also recoded on said syringe peripheral devices.

12. A liquid injection system according to any one of claims 1 through 6, further provided with patient peripheral devices including a wristband for accoutering around a wrist of said patient, and a form card for entering various kinds of data items about said patient, wherein
the two-dimensional codes representing various kinds of data items established in connection to each of said patient peripheral devices are also recoded on said patient peripheral devices.

13. A liquid injection system according to any one of claims 1 through 6, further provided with a liquid-temperature retaining apparatus for keeping the liquid in liquid syringe to be deployed at a predetermined temperature with a heat-retaining mechanism, separately from said liquid injector, wherein said liquid-temperature retaining apparatus is also provided with
a code-reading means for optically reading said two-dimensional codes,
a code-decoding means for decoding said two-dimensional codes that have been optically read, and
an operation-controlling means for executing predetermined operations corresponding to the decoded results of said two-dimensional codes.

14. A liquid injector 100 provided with
a code-reading means 122 for optically reading two-dimensional codes 214,
a code-decoding means 141 for decoding said two-dimensional codes 214 that have been optically read, and
an operation-controlling means 142 for executing predetermined operations corresponding to the decoded results of said two-dimensional codes 214, wherein
said liquid injector 100 is adapted to the liquid injection system 1000 according to any one of claims 1 through 12.

15. A liquid-temperature retaining apparatus provided with
a code-reading means 122 for optically reading two-dimensional codes 214,
a code-decoding means 141 for decoding said two-dimensional codes 214 that have been optically read, and
an operation-controlling means 142 for executing predetermined operations corresponding to the decoded results of said two-dimensional codes 214, wherein
said liquid-temperature retaining apparatus is adapted to the liquid injection system 1000 according to claim 13.
